# EUROPÄISCHE PATENTSCHRIFT

(11) **EP 0 308 843 B1**
(45) Veröffentlichungstag und Bekanntmachung des Hinweises auf die Patenterteilung: **15.12.1993**
(21) Anmeldenummer: 88115299.5
(22) Anmeldetag: 17.09.1988
(51) Int. Cl.: C07D 471/08, A61K 31/435

(54) **Bispidinderivate als Klasse III-Antiarrhythmika**
Bispidin derivatives as class III antiarrhythmics
Dérivés de bispidine comme antiarythmiques de classe III

(30) Priorität: 24.09.1987 DE 3732094
(43) Veröffentlichungstag der Anmeldung: 29.03.1989
(73) Patentinhaber: BASF Aktiengesellschaft, 67063 Ludwigshafen (DE)
(72) Erfinder: Lubisch, Wilfried, Dr., D-6800 Mannheim 1 (DE); Binnig, Fritz, Dr., D-6701 Fussgoenheim (DE); Von Philipsborn, Gerda, Dr., D-6940 Weinheim (DE)

(56) Entgegenhaltungen:
- EP-A- 0 062 199
- FR-A- 2 375 235

## Beschreibung

Die Erfindung betrifft neue Bispidinderivate, sie enthaltende Arzneimittel und deren Verwendung zur Herstellung von Antiarrhythmika der Klasse III nach Vaughan-Williams.

Die Antiarrhythmika lassen sich auf Grund der Klassifizierung von Vaughan-Williams in 4 Gruppen einteilen: I. Natrium-Antagonisten, II. adrenerge β-Rezeptorenblocker, III. Kalium-Kanal-Hemmstoffe, IV. Calcium-Antagonisten.

Bispidinderivate sind als Antiarrhythmika bekannt (Peter C. Ruenitz und Corwin M. Mokler, J. Med. Chem., 22, 1142 (1979), EP-A-62 199; DE-A-27 26 571). Sie gehören aufgrund ihres Wirkungsmechanismus zum größten Teil als Natrium-Antagonisten zur Klasse I nach Vaughan-Williams. Ambasilide (EP-A-62 199) weist dagegen zusätzlich eine Klasse-III-Wirkung auf.

Antiarrhythmika der Klasse III sind in der Therapie oft vorzuziehen, da sie bei sonst therapieresistenten Arrythmien wirken. Klasse-III-Antiarrhythmika führen zu Verlängerung der QT-Strecke im EKG, ohne die PQ-Dauer zu beeinflussen und ohne stärkere Herzfrequenzsenkung.

Solche sind bekannt beispielsweise aus EP-A-164 865, EP-A-178 874 und EP-A-158 775.

Es wurde nun gefunden, daß Bispidinderivate der Formel I 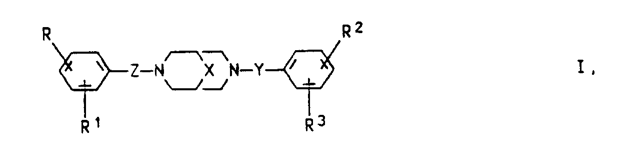
worin
- R, R¹ und R³: gleich oder verschieden sind und H, C₁-C₄-Alkyl, Halogen oder C₁-C₄-Alkoxy bedeuten, und
- R²: wenn 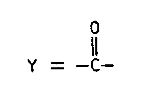 ist, C₁-C₄-Alkyl, Halogen, -CN, C₁-C₄-Alkoxy, -NHSO₂CH₃, -CF₃, NH-Acetyl und -NR⁴R⁵ bedeutet, wobei R⁴ und R⁵ C₁-C₄-Alkyl darstellen, und wenn 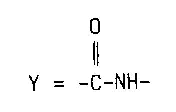 ist, C₁-C₄-Alkyl, Halogen, -CN, C₁-C₄-Alkoxy, -NHSO₂CH₃, -CF₃, NH-Acetyl und NR⁹R¹⁰ bedeuten, wobei R⁹ C₁-C₄-Alkyl und R¹⁰ H oder C₁-C₄-Alkyl bedeuten, und R², wenn nicht X = CH₂ ist, auch NO₂ und NH₂ sein kann,
- X: -CH₂-, -C(O)- oder -CHOH-,
- Y: -C(O)- oder -CONH- und
- Z: C₁-C₄-Alkylen, welches olefinisch ungesättigt und/oder verzweigt sein kann,
bedeuten, sowie deren physiologisch verträglichen Salze überlegene Eigenschaften besitzen.

R³ ist bevorzugt Wasserstoff, X -CH₂- und -CO-, Y -CONH- und Z Methylen.

Die erfindungsgemäßen Verbindungen können beispielsweise nach den folgenden Reaktionsschemata A bis C hergestellt werden:

### Reaktionsschema A:

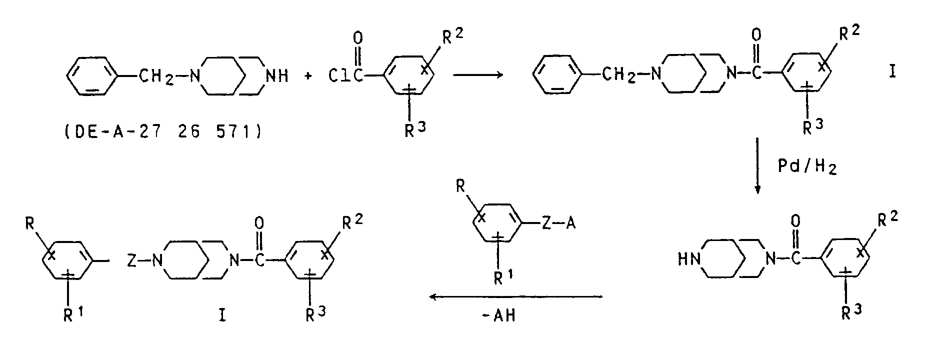

Das aus der DE-A 27 26 571 bekannte N-Monobenzylbispidin wird nach üblichen Methoden mit einem entsprechend substituierten Benzoylchlorid zum Amid umgesetzt, die Benzylgruppe hydrierend abgespalten und die entstandene sekundäre Aminogruppe mit einer Verbindung 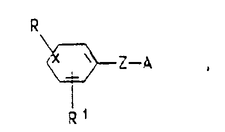
worin R, R¹ und Z die oben angegebene Bedeutung haben und A eine Austrittsgruppe darstellt, zum Endprodukt I umgesetzt. Die Austrittsgruppe A, die sich durch Nukleophile verdrängen läßt, kann beispielsweise sein ein Chlor- oder Bromatom, eine Methoxy- oder Ethoxygruppe, ein Oxisuccinimid-, 1-Imidazolyl- oder Ethoxycarbonyloxyrest.

### Reaktionsschema B:

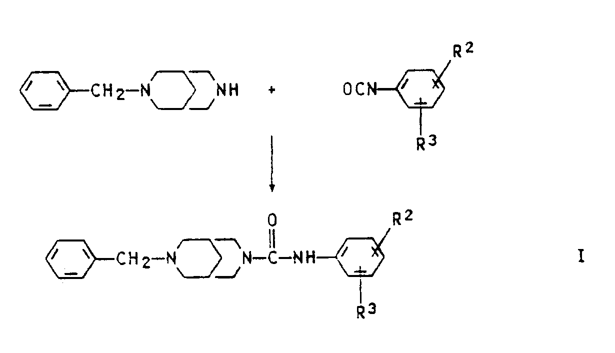

Dies ist eine von mehreren Möglichkeiten zur Herstellung von Verbindungen der allgemeinen Formel I mit einer Harnstoffgruppierung (vgl. Houben-Weyl, Meth. d. org. Ch., 4. Auflage, E4, S. 334ff). Bei der so erhaltenen Verbindung I, kann genau wie oben unter A beschrieben, der Benzylrest durch die Reste R und R¹ substituiert sein.

### Reaktionsschema C:

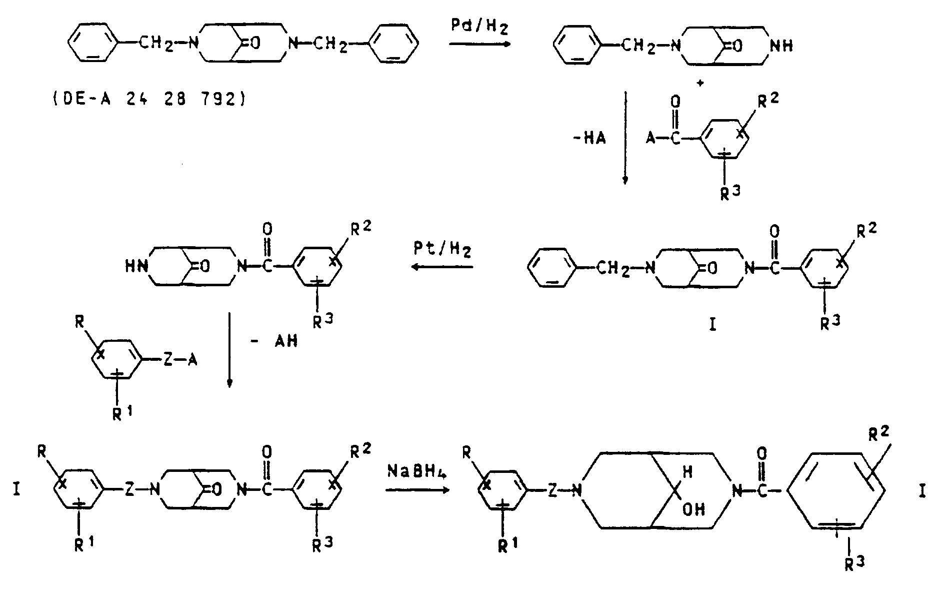

N,N'-Dibenzylbispidon wird partiell dehenzyliert (vgl. Bsp. 20), wie oben bei A benzoyliert und damit bereits in eine Verbindung der allgemeinen Formel I überführt. Hier kann nun wieder der Benzylrest gegen einen anderen Rest 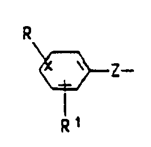
ausgetauscht werden, und die Carbonylgruppe kann z.B. mit Natriumboranat reduziert werden.

Falls bei den verschiedenen Synthesemöglichkeiten R² eine primare oder sekundäre Aminogruppe darstellt, kann diese beispielsweise durch Acetylierung geschützt und die Acetylgruppe nach weiteren Umsetzungen am Molekül hydrolytisch wieder abgespalten werden.

Gegebenenfalls werden die so erhaltenen Bispidinderivate in das Säureadditionssalz einer physiologisch verträglichen Säure überführt. Eine Zusammenstellung üblicher physiologisch verträglicher Säuren kann aus Fortschritte der Arzneimittelforschung, 1966, Birkhäuser Verlag. Bd. 10, S. 224 bis 285, Deutschland, Schweiz entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, oder einem niederen Keton, wie Aceton, Methylethylketon oder Methylisobutylketon, oder einem Ether, wie Diethylether, Tetrahydrofuran oder Dioxan erhalten. Zur besseren Kristallabscheidung können Mischungen der genannten Lösungsmittel verwendet werden. Darüber hinaus können pharmazeutisch vertretbare wäßrige Lösungen von Säure-Additionsverbindungen der Bispidinderivate der Formel I durch Auflösen der freien Basen in einer wäßrigen Säurelösung hergestellt werden.

Die Erfindung betrifft weiter therapeutische Mittel zur topischen und vor allem systemischen Anwendung, die eine Verbindung der Formel (I) neben üblichen Trägerstoffen oder Verdünnungsmitteln als Wirkstoff enthalten, und die Verwendung einer Verbindung der Formel (I) zur Herstellung eines Arzneimittels, insbesondere eines Antiarrhythmikums.

Die neuen Verbindungen besitzen, wie der folgende Versuch zeigt, eine gute antiarrhythmische Klasse-III-Wirkung:
Als Versuchstiere dienen männliche und weibliche Pirbright-white-Meerschweinchen im Gewicht von 300 bis 500 g. Die Narkose erfolgt mit 1,5 g/kg Urethan i.p. Die Substanzen werden intravenös appliziert. Zur Messung der EKG-Leitungszeiten und der Herzfrequenz wird die II. Extremitätenableitung registriert. Meßgröflen sind die QT- und die PQ-Strecken und die Herzfrequenz. Pro Dosis werden 4 bis 6 Tiere eingesetzt. Kriterium für Klasse-III-Wirkung ist Zunahme der QT-Dauer im Vergleich zu den Werten vor Substanzgabe. PQ-Zunahme und starke Herzfrequenzabnahme dienen als Ausschlußkriterien. Aus der linearen Beziehung zwischen log Dosis (mg/kg) der Substanz und der relativen Verlangerung der QT-Dauer (Δ %) wird die ED 20 % berechnet.

**Tabelle 1**

| Antiarrhythmische Klasse-III-Wirkung beim Meerschweinchen nach intravenöser Applikation. | |
|---|---|
| Beispiel Nr. | Verlängerung der QT-Dauer ED 20 % [mg/kg] Mittelwert; |
| 2 | 4,6 |
| 14 | 2,6 |
| 16 | 2,4 |
| 19 | 3,6 |
| Ambasilide | 6,3 |
| D-Sotalol | 3,6 |

Die erfindungsgemäßen Substanzen (Tabelle 1) sind hinsichtlich QT-Verlängerung wirksamer als das Antiarrhythmikum Ambasilide und zum Teil auch dem als Klasse-III-Antiarrhythmikum bekannten D-Sotalol (Clin. Sci., 69, 631-636 (1985); J. Cardiovascul. Pharmacol., 6, 1132-1141 (1984)) überlegen.

Die neuen Substanzen eignen sich daher zur Behandlung sonst therapieresistenter Arrhythmien, insbesondere beseitigen sie ventrikuläre Tachykardien, die nach Myokard-Infarkt auftreten und auf einem "re-entry"-Mechanismus beruhen (Lit. Cardiac Arrhythmia Ed. P. Brugada, H.J.J. Wellens, Futura Publishing Co., Mount Kisko, New York 1987).

Die Herstellung der therapeutischen Mittel oder Zubereitungen erfolgt mit den üblichen flüssigen oder festen Trägerstoffen oder Verdünnungsmitteln und den in üblicher Weise verwendeten pharmazeutisch-technischen Hilfsstoffen, entsprechend der gewünschten Applikationsart und mit einer zur Anwendung geeigneten Dosierung, in üblicher Weise, beispielsweise durch Vermischen des Wirkstoffes mit den an sich in solchen Präparaten üblichen festen und flüssigen Träger- und Hilfsstoffen.

Die Mittel können peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen Mittel können die erfindungsgemäß zu verwendenden Verbindungen bei lokaler Anwendung in 0,0001 bis 1 %iger Konzentration, bevorzugt in 0,001 bis 0,1 %iger Konzentration und bei systemischer Anwendung vorzugsweise in einer Einzeldosis von 0,1 bis 20 mg pro kg-Gew, und zwar parenteral 0,1 bis 4 und oral 1 bis 20 mg pro kg Körpergewicht, enthalten und täglich in einer oder mehreren Dosierungen je nach Art und Schwere der Erkrankungen verabreicht werden. Dementsprechend enthalten die Arzneimittel zur parenteralen Anwendung pro Einzeldosis 5 bis 200 mg, zur oralen Anwendung 50 bis 1000 mg des erfindungsgemäßen Wirkstoffs.

Üblicherweise verwendete pharmazeutisch technische Hilfsstoffe sind beispielsweise für die lokale Anwendung Alkohole, wie Ethanol, Isopropanol, oxethyliertes Ricinusöl oder oxethyliertes hydriertes Ricinusöl, Polyacrylsäure, Glycerinmonostearat, Paraffinöl, Vaseline, Wollfett, Polyethylenglykol, Polypropylenglykol, Stearat sowie ethoxylierter Fettalkohol. für die systemische Anwendung Milchzucker, Propylenglykol und Ethanol, Stärke, Talk, Polyvinylpyrrolidon. Den Präparaten kann gegebenenfalls ein Antioxidationsmittel, beispielsweise Tocopherol sowie butyliertes Hydroxyanisol oder butyliertes Hydroxytoluol oder geschmacksverbessernde Zusätze, Stabilisierungsmittel, Emulgiermittel, Bleichmittel usw. zugesetzt werden. Voraussetzung ist, daß alle bei der Herstellung pharmazeutischer Zubereitungen verwendeten Stoffe toxikologisch unbedenklich und mit den verwendeten Wirkstoffen verträglich sind.

### Beispiel 1

### 3-(4-Acetaminobenzoyl)-7-benzyl-3,7-diazabicyclo[3.3.1]nonan

80,2 g (0,24 mol) 3-(4-Aminobenzoyl)-7-benzyl-3,7-diazabicyclo[3.3.1]nonan (vgl. EP 62 199) wurden in 500 ml wasserfreiem Tetrahydrofuran gelöst und mit Eis gekühlt. Dazu tropfte man 23.5 g (0,3 mol) Acetylchlorid, gelöst in 50 ml Tetrahydrofuran, und anschließend bei Raumtemperatur noch 48,6 g (0,48 mol) Triethylamin. Man ließ alles über Nacht rühren. Anschließend wurde im Vakuum eingeengt. Der Rückstand wurde in Wasser aufgenommen und 3 x mit Methylenchlorid extrahiert. Die vereinigten organischen Phasen wurden mit Wasser gewaschen, getrocknet und im Vakuum eingeengt. Ausbeute: 78,1 g, Schmp.: 208°C.

### Beispiel 2

### 7-Benzyl-3-(4-chlorbenzoyl)-3,7-diaza-bicyclo[3.3.1]-nonanhydrochlorid

5 g (23 mmol) des Monobenzylbispidins wurden in 100 ml Methylenchlorid gelöst und mit 20 ml 2 M NaOH versetzt. Bei Eiskühlung und kräftigem Rühren tropfte man 4,9 g (28 mmol) 4-Chlorbenzoylchlorid, gelöst in 10 ml Methylenchlorid, zu. Man ließ 1 h rühren, verdünnte mit Wasser und isolierte die Methylenchlorid-Phase, die danach noch mit Wasser gewaschen, getrocknet und mit etherischem HCl versetzt wurde. Das erhaltene Öl kristallisierte aus Essigester/Ether. Ausbeute 4,9 g. Schmp. 166°C (x2HCl).

Analog Beispiel 2 wurden folgende Verbindungen hergestellt:
3. 7-Benzyl-3-(2-methoxybenzoyl)-3,7-diaza-bicyclo[3.3.1]-nonanhydrochlorid, Schmp. 162°C (xHCl).
4. 7-Benzyl-3-(2-chlorbenzoyl)-3,7-diaza-bicyclo[3.3.1]-nonan, Schmp. 266°C (x2HCl).
5. 7-Benzyl-3-[4-dimethylaminobenzoyl)-3,7-diaza-bicyclo[3.3.1]-nonan, Schmp. 66°C (x2HCl).
6. 7-Benzyl-3-(4-methoxybenzoyl)-3,7-diazabicyclo[3.3.1]-nonan, Schmp. 119°C (xHCl).
7. 7-Benzyl-3-(4-cyanobenzoyl)-3,7-diazabicyclo[3.3.1]-nonan, Schmp.: 170°C (x2HCl).
8. 7-Benzyl-3-(2-methylbenzoyl)-3,7-diazabicyclo[3.3.1]-nonan, Schmp.: 243°C (xHCl).
9. 7-Benzyl-3-(4-brombenzoyl)-3,7-diazabicyclo[3.3.1]-nonan, Schmp.: 166°C (x2HCl).
10. 7-Benzyl-3-(4-methylbenzoyl)-3,7-diazabicyclo[3.3.1]-nonan, Schmp. 133°C (x2HCl).
11. 7-Benzyl-3-(4-fluorbenzoyl)-3,7-diazabicyclo[3.3.1]-nonan, Schmp.: 133°C (x2HCl).
12. 7-(Benzyl-3-(4-trifluormethylbenzoyl)-3,7-diazabicyclo[3.3.1]-nonan. Schmp. 89°C.

### Beispiel 13

### 7-Benzyl-3-(4-methansulfonylamidobenzoyl)-3,7-diazabicyclo[3.3.1]nonan

Zu 5,0 g (14,9 mmol) des 3-(4-Aminobenzoyl)-7-benzyl-3,7-diazabicyclo[3.3.1]nonans, das in 50 ml Pyridin gelöst wurde, tropfte man bei 5 bis 10°C 1,9 g (16,4 mmol) Methansulfonylchlorid. Es wurde noch 16 h bei der Temperatur gerührt. Das Reaktionsgemisch wurde auf 200 ml Wasser gegossen, wobei sich das Produkt abschied, das durch Abdekantieren vom Wasser befreit wurde. Das erhaltene Produkt wurde aus Essigester umkristallisiert. Ausbeute 4,1 g, Schmp. 172°C.

### Beispiel 14

### 7-Benzyl-3-(4-methylphenylcarbamoyl)-3,7-diazabicyclo[3.3.1]nonan

2,6 g (0,012 mol) Monobenzylbispidin, 1,6 g (0,012 mol) 4-Methylphenylisocyanat und 0,25 ml Triethylamin wurden in 25 ml Ligroin gelöst und 1 h auf Rückflußtemperatur erwärmt. Der entstandene Niederschlag wurde isoliert und aus Ligroin/2-Propanol umkristallisiert. Ausbeute: 2,5 g, Schmp.: 150°C.

Analog Beispiel 14 wurden folgende Verbindungen aus Monobenzylbispidin hergestellt:
15. 7-Benzyl-3-(4-methoxyphenylcarbamoyl)-3,7-diaza-bicyclo[3.3.1]nonan, Schmp.: 161°C.
16. 7-Benzyl-3-(4-chlorphenylcarbamoyl)-3,7-diaza-bicyclo[3.3.1]nonan, Schmp.: 175°C.
17. 7-Benzyl-3-(phenylcarbamoyl)-3,7-diaza-bicyclo[3.3.1]nonan, Öl.

### Beispiel 18

### 3-(4-Aminophenylcarbamoyl)-7-benzyl-3,7-diaza-bicyclo[3.3.1]nonan

5,2 g (24 mmol) Monobenzylbispidin, 3,95 g (24 mmol) 4-Nitrophenylisocyanat und 0,50 ml Triethylamin wurden in 50 ml Ligroin zusammengegeben und anschließend für 1 h auf Rückflußtemperatur erwärmt. Man isolierte den entstandenen Niederschlag und kristallisierte aus Methanol/Isopropanol um. Ausbeute: 6,7 g.

5,6 g (14,7 mmol) der so erhaltenen Nitro-Verbindung wurden in 100 ml Methanol aufgenommen, mit 0,6 g Platin/Kohle (5 %ig) versetzt und bei RT und 1 bar hydriert. Anschließend wurde filtriert und das Filtrat im Vakuum eingeengt. Ausbeute 4,5 g, Schmp.: 77°C.

### Beispiel 19

### 3-(Phenylcarbamoyl)-7-(4-chlorbenzyl)-3,7-diaza-bicyclo[3.3.1]nonan

18,7 g (55,7 mmol) 7-Benzyl-3-(phenylcarbamoyl)-3,7-diazabicyclo[3.3.1]nonans wurden in 170 ml Methanol und 30 ml Eisessig gelöst. Man versetzte mit 1,1 g Palladium/Kohle (10 %ig) und hydrierte bei Raumtemperatur bis zum equimolaren Wasserstoffverbrauch. Anschließend wurde filtriert, das Filtrat mit Natronlauge alkalisiert und im Vakuum eingeengt. Der Rückstand wurde 3x mit Methylenchlorid extrahiert. Die so erhaltene organische Phase wurde getrocknet und im Vakuum eingeengt. Man kristallisierte aus Essigester um. Ausbeute 8,7 g.

3,5 g (14,3 mmol) des so erhaltenen Amins, 3,2 g (20,0 mmol) 4-Chlorbenzylchlorid und 5 ml Triethylamin wurden in 50 ml Methanol gelöst und für 60 h bei Raumtemperatur gerührt. Anschließend erwärmte man für 2 h auf 60°C. Das Reaktionsgemisch wurde danach mit Natronlauge schwach alkalisch gestellt und im Vakuum eingeengt. Der Rückstand wurde 3x mit Methylenchlorid extrahiert, die organische Phase getrocknet und im Vakuum eingeengt. Man kristallisierte aus Essigester um. Ausbeute 2,0 g, Schmp.: 161°C.

### Beispiel 20

### 7-Benzyl-3-(4-chlorbenzoyl)-3,7-diazabicyclo[3.3.1]-9-nonon

32,0 g (0,1 mol) 3,7-Dibenzyl-3,7-diazabicyclo[3.3.1]-9-nonon wurden in 250 ml Methanol gelöst, mit 1 g Palladium/Kohle (10 %ig) versetzt und bis zum equimolaren Wasserstoffverbrauch hydriert. Man filtrierte anschließend und engte das Filtrat im Vakuum ein. Man erhielt 14,1 g 3-Benzyl-3,7-diazabicyclo[3.3.1]-9-nonon. Schmp. 110°C.

14,0 g (60,9 mmol) des so erhaltenen Produkts wurden in 100 ml Methylenchlorid gelöst, mit 100 ml 2 M Natronlauge versetzt und eisgekühlt. Man ließ 16,0 g (91,3 mmol) 4-Chlorbenzoylchlorid, in Methylenchlorid gelöst, zutropfen und weitere 3 min rühren. Die organische Phase wurde abgetrennt, mit Natronlauge und Wasser gewaschen, getrocknet und im Vakuum eingeengt. Ausbeute: 22 g, Schmp.: 154°C (xHCl).

### Beispiel 21

### 7-Benzyl-3-(4-chlorbenzoyl)-3,7-diazabicyclo[3.3.1]-9-nonol

14,2 g (38,5 mmol) des nach Beispiel 20 erhaltenen Ketons wurden in 150 ml Methanol gelöst und portionsweise mit 1,5 g (39,5 mmol) Natriumborhydrid versetzt. Man ließ noch 1 h bei RT rühren und tropfte dann 70 ml 1 M Salzsäure zu. Die Lösung wurde im Vakuum eingeengt, der Rückstand in Methylenchlorid gelöst, mit Natronlauge und Wasser gewaschen, getrocknet und eingeengt. Ausbeute: 14 g, Schmp.: 167°C.

### Beispiel 22

### 7-Benzyl-3-(4-nitrobenzoyl)-3,7-diazabicyclo[3.3.1]-9-nonon

11,3 g (49,1 mmol) 7-Benzyl-3,7-diazabiyclo[3.3.1]-9-nonon wurden in 100 ml Methylenchlorid gelöst und mit 100 ml 2 M Natronlauge versetzt. Bei 0°C tropfte man eine Lösung aus 13,7 g (73.7 mmol) 4-Nitrobenzoylchlorid in 30 ml Methylenchlorid zu und ließ noch 1 h rühren. Die organische Phase wurde abgetrennt, mit Natronlauge und Wasser gewaschen, getrocknet und eingeengt. Der Rückstand wurde aus Aceton umkristallisiert. Man erhielt 10,5 g, Schmp. 138°C.

### Beispiel 23

### 7-Benzyl-3-(4-nitrobenzoyl)-3,7-diazabicyclo[3.3.1]-9-nonol

15,0 g (39,6 mmol) des nach Beispiel 22 erhaltenen Ketons wurden in 150 ml Methanol gelöst. Bei Raumtemperatur gab man 1,5 g (39,6 mmol) Natriumborhydrid portionsweise zu und ließ noch 1 h rühren. Danach tropfte man 50 ml 2 M Salzsäure zu. ließ 2 h bei Raumtemperatur rühren und engte das Gemisch im Vakuum ein. Der Rückstand wurde zwischen Methylenchlorid und Natronlauge verteilt, die organische PHase separiert, nochmals mit Natronlauge, dann mit Wasser gewaschen, getrocknet und eingeengt. Man kristallisierte aus Essigester und erhielt 11,5 g, Schmp.: 210°C.

Ausgehend von Beispiel 22 wurden durch katalytische Hydrierung hergestellt:
24. 3-(4-Aminobenzoyl)-7-benzyl-3,7-diazabicyclo[3.3.1]-9-nonon, Schmp.: 235°C (xHCl).
25. 3-(4-Aminobenzoyl)-7-benzyl-3,7-diazabicyclo[3.3.1]-9-nonol. Schmp. 232°C (x2HCl).

## Patentansprüche

1. Bispidinderivate der Formel I 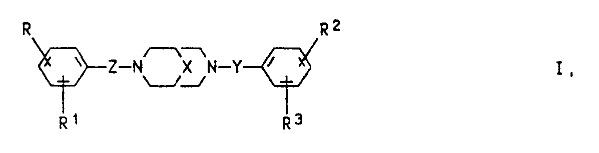 worin
R, R¹ und R³ gleich oder verschieden sind und H, C₁-C₄-Alkyl. Halogen oder C₁-C₄-Alkoxy bedeuten, und
R² wenn 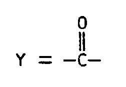 ist, C₁-C₄-Alkyl, Halogen, -CN, C₁-C₄-Alkoxy, -NHSO₂CH₃, -CF₃, NH-Acetyl und -NR⁴R⁵ bedeutet, wobei R⁴ und R⁵ C₁-C₄-Alkyl darstellen, und wenn 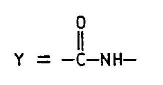 ist, C₁-C₄-Alkyl, Halogen, -CN, C₁-C₄-Alkoxy, -NHSO₂CH₃, -CF₃, NH-Acetyl und -NR⁹R¹⁰ bedeuten, wobei R⁹ C₁-C₄-Alkyl und R¹⁰ H oder C₁-C₄-Alkyl bedeuten, und R², wenn nicht X = CH₂ ist, auch NO₂ und NH₂ sein kann.
X -CH₂-, -C(O)- oder -CHOH -,
Y -C(O)- oder -CONH- und
Z C₁-C₄-Alkylen, welches olefinisch ungesättigt und/oder verzweigt sein kann,
bedeuten, sowie deren physiologisch verträglichen Salze.

2. Bispidinderivate nach Anspruch 1, dadurch gekennzeichnet, daß Y den Rest -CONH- darstellt.

3. 7-Benzyl-3-(phenylcarbamoyl)-3,7-diaza-bicyclo[3.3.1]nonan; 3-(4-Aminophenylcarbamoyl)-7-benzyl-3,7-diaza-bicyclo[3.3.1]nonan und 3-(phenylcarbamoyl)-7-(4-chlorbenzyl)-3,7-diaza-bicyclol[3.3.1]nonan.

4. Arzneimittel, das neben üblichen Hilfsstoffen als Wirkstoff ein Bispidinderivat nach Anspruch 1 enthält.

5. Arzneimittel zur topischen Anwendung, das 0,0001 bis 1 Gew.% eines Bispidinderivates nach Anspruch 1 neben üblichen Hilfsstoffen enthält.

6. Arzneimittel zur parenteralen Anwendung, das pro Einzeldosis 5 bis 200 mg eines Bispidinderivates nach Anspruch 1 neben üblichen Hilfsstoffen enthält.

7. Arzneimittel zur oralen Anwendung, das pro Einzeldosis 50 bis 1000 mg eines Bispidinderivates nach Anspruch 1 neben üblichen Hilfsstoffen enthält.

8. Verwendung einer Verbindung nach Anspruch 1 zur Herstellung eines Arzneimittels.

9. Verwendung einer Verbindung nach Anspruch 1 zur Herstellung eines Antiarrhythmikums der Klasse III nach Vaughan-Williams.

## Claims

1. A bispidine derivative of the formula I 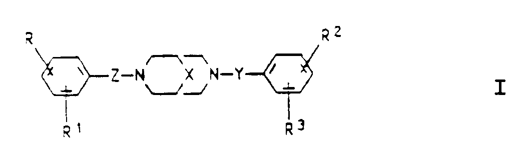 where R, R¹ and R³ are identical or different and are each H, C₁-C₄-alkyl, halogen or C₁-C₄-alkoxy, when Y is 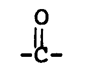 R² is C₁-C₄-alkyl, halogen, -CN, C₁-C₄-alkoxy, -NHSO₂CH₃, -CF₃, NH-acetyl or -NR⁴R⁵, where R⁴ and R⁵ are each C₁-C₄-alkyl, when Y is 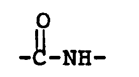 R² is C₁-C₄-alkyl, halogen, -CN, C₁-C₄-alkoxy, -NHSO₂CH₃, -CF₃, NH-acetyl or -NR⁹R¹⁰, where R⁹ is C₁-C₄-alkyl and R¹⁰ is H or C₁-C₄-alkyl, and when X is not CH₂ R₂ may also be NO₂ or NH₂, X is -CH₂-, -C(O)- or -CHOH-, Y is -C(O)- or -CONH- and Z is C₁-C₄-alkylene which may be olefinically unsaturated and/or branched, and its physiologically tolerated salts.

2. A bispidine derivative as claimed in claim 1, wherein Y is -CONH-.

3. 7-Benzyl-3-(phenylcarbamoyl)-3,7-diazabicyclo[3.3.1]nonane, 3-(4-aminophenylcarbamoyl)-7-benzyl-3,7-diazabicyclo[3.3.1]nonane and 3-(phenylcarbamoyl)-7-(4-chlorobenzyl)-3,7-diazabicyclo[3.3.1]nonane.

4. A drug which contains as the active compound a bispidine derivative as claimed in claim 1, in addition to conventional auxiliaries.

5. A drug for topical administration, which contains from 0.0001 to 1% by weight of a bispidine derivative as claimed in claim 1, in addition to conventional auxiliaries.

6. A drug for parenteral administration, which contains, per single dose, from 5 to 200 mg of a bispidine derivative as claimed in claim 1, in addition to conventional auxiliaries.

7. A drug for oral administration, which contains, per single dose, from 50 to 1,000 mg of a bispidine derivative as claimed in claim 1, in addition to conventional auxiliaries.

8. The use of a compound as claimed in claim 1 for the preparation of a drug.

9. The use of a compound as claimed in claim 1 for the preparation of an antiarrhythmic agent of class III according to Vaughan-Williams.

## Revendications

1. Dérivés de bispidine de formule I 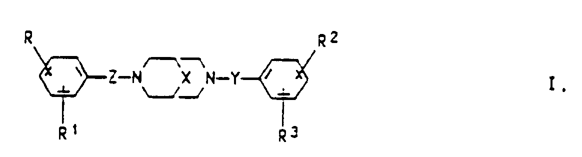 dans laquelle
R, R¹ et R³ sont identiques ou différents et représentent chacun un atome d'hydrogène, un reste alkyle en C₁-C₄, un atome d'halogène ou un reste alcoxy en C₁-C₄, et
R² représente, quand 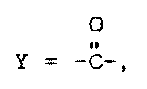 un reste alkyle en C₁-C₄, un atome d'halogène, -CN, un reste alcoxy en C₁-C₄, -NHSO₂CH₃, -CF₃, un groupement NH-acétyle ou -NR⁴R⁵, R⁴ et R⁵ étant des restes alkyle en C₁-C₄, et quand 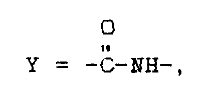 il représente un reste alkyle en C₁-C₄, un atome d'halogène, -CN, un reste alcoxy en C₁-C₄, -NHSO₂CH₃, -CF₃ un groupement NH-acétyle ou -NR⁹R¹⁰, R⁹ étant un reste alkyle en C₁-C₄ et R¹⁰ étant un atome d'hydrogène ou un reste alkyle en C₁-C₄, et R² peut aussi représenter NO₂ ou NH₂ quand X n'est pas mis pour CH₂,
X est mis pour -CH₂-, -C(O)- ou -CHOH-,
Y est mis pour -C(O)- ou -CONH- et
Z est un reste alkylène en C₁-C₄ qui peut présenter une insaturation oléfinique et/ou ètre ramifié,
ainsi que leurs sels acceptables physiologiquement.

2. Dérivés de bispidine selon la revendication 1, caractérisés en ce que Y représente le reste -CONH-.

3. 7-Benzyl-3-(phénylcarbamoyl)-3,7-diazabicyclo[3.3.1]nonane; 3-(4-aminophénylcarbamoyl)-7-benzyl-3,7-diazabicyclo[3.3.1]nonane et 3-(phénylcarbamoyl)-7-(4-chlorobenzyl)-3,7-diazabicyclo[3.3.1]nonane.

4. Médicament qui contient, comme substance active, un dérivé de bispidine selon la revendication 1, en dehors d'excipients ou d'adjuvants usuels.

5. Médicament à usage topique qui contient de 0,0001 à 1% en poids d'un dérivé de bispidine selon la revendication 1, en dehors d'excipients ou d'adjuvants usuels.

6. Médicament à usage par voie parentérale qui contient, par unité posologique, de 5 à 200 mg d'un dérivé de bispidine selon la revendication 1, en dehors d'excipients ou d'adjuvants usuels.

7. Médicament à usage par voie orale qui contient, par unité posologique, de 50 à 1000 mg d'un dérivé de bispidine selon la revendication 1, en dehors d'excipients ou d'adjuvants usuels.

8. Utilisation d'un composé selon la revendication 1 pour le préparation d'un médicament.

9. Utilisation d'un composé selon la revendication 1 pour le préparation d'un antiarythmique de la classe III dans la classification de Vaugham-Williams.
